(19) Europäisches Patentamt
European Patent Office
Office européen des brevets

(11) **EP 4 215 117 B1**

(12) **EUROPEAN PATENT SPECIFICATION**

(45) Date of publication and mention
of the grant of the patent:
**30.10.2024 Bulletin 2024/44**

(51) International Patent Classification (IPC):
**A61B 6/00** *(2024.01)*     **A61B 6/02** *(2006.01)*
**A61B 6/50** *(2024.01)*

(21) Application number: **23151926.5**

(52) Cooperative Patent Classification (CPC):
**A61B 6/502; A61B 6/025; A61B 6/5217**

(22) Date of filing: **17.01.2023**

(54) **IMAGE PROCESSING APPARATUS, IMAGE PROCESSING METHOD, AND PROGRAM**

BILDVERARBEITUNGSVORRICHTUNG, BILDVERARBEITUNGSVERFAHREN UND PROGRAMM

APPAREIL DE TRAITEMENT D'IMAGE, PROCÉDÉ DE TRAITEMENT D'IMAGE ET PROGRAMME

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **19.01.2022 JP 2022006671**

(43) Date of publication of application:
**26.07.2023 Bulletin 2023/30**

(73) Proprietor: **FUJIFILM Corporation**
**Tokyo 106-8620 (JP)**

(72) Inventor: **MACHII, Yusuke**
**Kanagawa, 258-8538 (JP)**

(74) Representative: **Dehns Germany Partnerschaft mbB**
**Theresienstraße 6-8**
**80333 München (DE)**

(56) References cited:
**US-A1- 2012 207 373**

- **WHEELER F W ET AL: "Micro-Calcification Detection in Digital Tomosynthesis Mammography", PROCEEDINGS OF SPIE, IEEE, US, vol. 6144, 13 February 2006 (2006-02-13), pages 614420 - 1, XP002497881, ISBN: 978-1-62841-730-2, DOI: 10.1117/12.653478**
- **JEONG JI-WOOK ET AL: "Simplified computer-aided detection scheme of microcalcification clusters in digital breast tomosynthesis images", 2016 38TH ANNUAL INTERNATIONAL CONFERENCE OF THE IEEE ENGINEERING IN MEDICINE AND BIOLOGY SOCIETY (EMBC), IEEE, 16 August 2016 (2016-08-16), pages 1070 - 1073, XP032979340, DOI: 10.1109/EMBC.2016.7590888**
- **RAVI K SAMALA ET AL: "Computer-aided detection system for clustered microcalcifications in digital breast tomosynthesis using joint information from volumetric and planar projection images", PHYSICS IN MEDICINE AND BIOLOGY, INSTITUTE OF PHYSICS PUBLISHING, BRISTOL GB, vol. 60, no. 21, 14 October 2015 (2015-10-14), pages 8457 - 8479, XP020290381, ISSN: 0031-9155, [retrieved on 20151014], DOI: 10.1088/0031-9155/60/21/8457**

**Description**

BACKGROUND OF THE INVENTION

1. Field of the Invention

The present disclosure relates to an image processing apparatus, an image processing method, and a program.

2. Description of the Related Art

A technique for recognizing a tissue of a breast in which calcification may occur by using a radiation image obtained by irradiating the breast with radiations is known. For example, JP2016-022143A discloses a technique of specifying pixel regions in which calcification may occur from a radiation image or the like, grouping a set of the specified pixel regions, and displaying the set of the specified pixel regions in color or brightness according to the number of the pixel regions belonging to the group. Thereby, it is possible to intuitively recognize a dense state of a fine calcification tissue.

In addition, tomosynthesis imaging in which a series of a plurality of projection images is acquired by irradiating a breast with radiations having a plurality of angles is known. By reconfiguring the plurality of projection images obtained by tomosynthesis imaging, a plurality of tomographic images in which an overlap of mammary glands is reduced are obtained. Further, a technique of generating one synthesized two-dimensional image in which an overlap of mammary glands is reduced by combining a plurality of tomographic images is known. In addition, JP2020-141867A discloses a technique of generating a two-dimensional image corresponding to the synthesized two-dimensional image by inputting a projection image obtained at a radiation irradiation angle of approximately 0 degree to a learned model instead of the plurality of tomographic images.

F W Wheeler et al. disclose in "Micro-Calcification Detection in Digital Tomosynthesis Mammography" (PROCEEDINGS OF SPIE, IEEE, US, vol. 6144, 13 February 2006, pages 614420-1) a technique for the detection and enhancement of microcalcifications in digital tomosynthesis mammography. Calcification residual images are computed for each of the projection images and calcification detection is performed over 3D space, based on the values of the calcification residual images at projection points for each 3D point under test.

SUMMARY OF THE INVENTION

In image diagnosis for diagnosing calcification of a breast, a shape of a calcification image appearing in a tomographic image, a synthesized two-dimensional image, or the like is important information. However, in the tomographic image, the calcification image is blurred due to noise and visibility is lowered. In addition, in the synthesized two-dimensional image generated based on the plurality of tomographic images, a shape of the calcification image is not accurately represented.

In JP2016-022143A, a distribution state of the calcification image is considered as useful information for image diagnosis for diagnosing calcification. However, a shape of the calcification image is not considered. Further, also in JP2020-141867A, a shape of the calcification image is not considered as useful information for image diagnosis for diagnosing calcification. In image diagnosis for diagnosing calcification, in order to determine whether the calcification image represents malignancy or benignancy, it is desired to accurately determine a type of a shape of the calcification image.

An object of a technique of the present disclosure is to provide an image processing apparatus, an image processing method, and a program capable of accurately determining a type of a shape of a calcification image.

In order to achieve the above object, according to an aspect of the present disclosure, there is provided an image processing apparatus including: at least one processor, in which the processor is configured to execute calcification image detection processing of detecting a calcification image based on a plurality of tomographic images obtained from a series of a plurality of projection images obtained by tomosynthesis imaging of a breast, region-of-interest image group generation processing of generating a region-of-interest image group by cutting out, as a region-of-interest image, a region including the calcification image detected by the calcification image detection processing from each of the plurality of projection images, variance value calculation processing of calculating a variance value of feature amounts of each of the region-of-interest images included in the region-of-interest image group, and shape type determination processing of determining a type of a shape of the calcification image based on the variance value calculated by the variance value calculation processing.

Preferably, the processor is configured to individually generate the region-of-interest image group for each of a plurality of the calcification images in the region-of-interest image group generation processing in a case where the plurality of calcification images are detected in the calcification image detection processing.

Preferably, the processor is configured to detect only the calcification image of which a signal value is equal to or smaller than a certain value in the calcification image detection processing.

Preferably, the processor is configured to determine a shape of the calcification image based on a relationship between a predetermined variance value and a type of a shape in the shape type determination processing.

Preferably, the feature amount is a variance value of pixel values included in one of the region-of-interest images.

Preferably, the feature amount is a variance value of pixel values included in one of the region-of-interest images with respect to an average value of pixel values in a breast region of one of the projection images.

Preferably, the feature amount is the number of pixels having a pixel value equal to or larger than a threshold value among a plurality of pixels included in one of the region-of-interest images.

Preferably, the processor is configured to further execute display processing of displaying a shape type determination result by the shape type determination processing on a display unit.

Preferably, the processor is configured to highlight and display the calcification image having a specific shape based on the shape type determination result in the display processing.

According to another aspect of the present disclosure, there is provided an image processing method including: a calcification image detection step of detecting a calcification image based on a plurality of tomographic images obtained from a series of a plurality of projection images obtained by tomosynthesis imaging of a breast; a region-of-interest image group generation step of generating a region-of-interest image group by cutting out, as a region-of-interest image, a region corresponding to the calcification image detected by the calcification image detection step from each of the plurality of projection images; a variance value calculation step of calculating a variance value of feature amounts of each of the region-of-interest images included in the region-of-interest image group; and a shape type determination step of determining a type of a shape of the calcification image based on the variance value calculated by the variance value calculation step.

According to still another aspect of the present disclosure, there is provided a program causing a computer to execute a process including: calcification image detection processing of detecting a calcification image based on a plurality of tomographic images obtained from a series of a plurality of projection images obtained by tomosynthesis imaging of a breast; region-of-interest image group generation processing of generating a region-of-interest image group by cutting out, as a region-of-interest image, a region corresponding to the calcification image detected by the calcification image detection processing from each of the plurality of projection images; variance value calculation processing of calculating a variance value of feature amounts of each of the region-of-interest images included in the region-of-interest image group; and shape type determination processing of determining a type of a shape of the calcification image based on the variance value calculated by the variance value calculation processing.

According to the technique of the present disclosure, it is possible to provide an image processing apparatus, an image processing method, and a program capable of accurately determining a type of a shape of a calcification image.

BRIEF DESCRIPTION OF THE DRAWINGS

Fig. 1 is a diagram illustrating an example of an entire configuration of a radiography system.
Fig. 2 is a diagram illustrating an example of tomosynthesis imaging.
Fig. 3 is a block diagram illustrating an example of a configuration of an image processing apparatus.
Fig. 4 is a block diagram illustrating an example of a function realized by a controller of the image processing apparatus.
Fig. 5 is a diagram schematically illustrating a flow of processing by the image processing apparatus.
Fig. 6 is a diagram conceptually illustrating an example of region-of-interest image group generation processing.
Fig. 7 conceptually illustrates an example of variance value calculation processing.
Fig. 8 is a diagram illustrating an example of display processing.
Fig. 9 is a flowchart illustrating a flow of a series of processing by the image processing apparatus.
Fig. 10 is a block diagram illustrating a function realized by the controller of the image processing apparatus according to a modification example.
Fig. 11 is a block diagram schematically illustrating a flow of processing by the image processing apparatus according to the modification example.

DESCRIPTION OF THE PREFERRED EMBODIMENTS

Hereinafter, an embodiment of the present disclosure will be described with reference to the drawings.

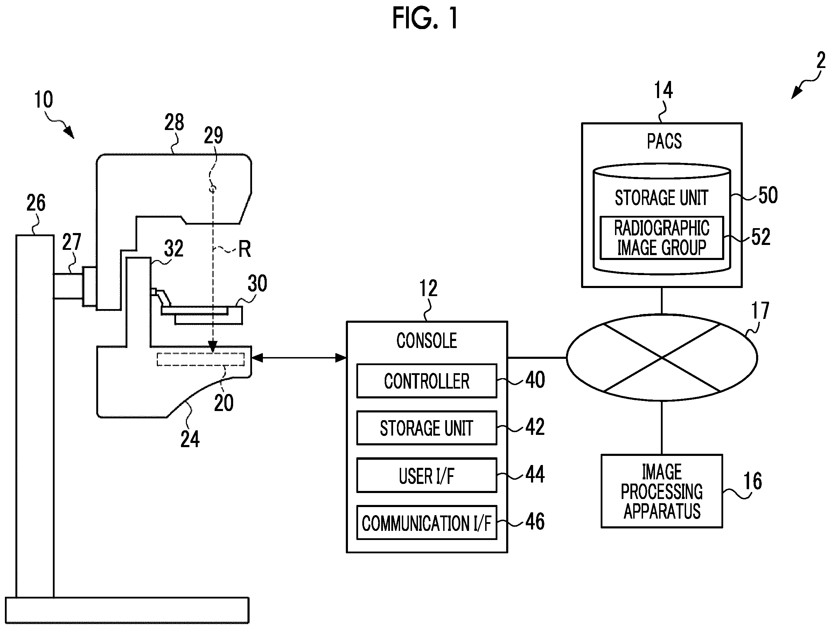

Fig. 1 illustrates an example of an entire configuration of a radiography system 2 according to the present embodiment. The radiography system 2 includes a mammography apparatus 10, a console 12, a picture archiving and communication systems (PACS) 14, and an image processing apparatus 16. The console 12, the PACS 14, and the image processing apparatus 16 are connected to each other via a network 17 by wired communication or wireless communication.

Fig. 1 illustrates an example of an appearance of the mammography apparatus 10. Fig. 1 illustrates an example of an appearance in a case where the mammography apparatus 10 is viewed from a left side of a subject.

The mammography apparatus 10 operates according to a control of the console 12, and is a radiography apparatus that acquires a radiation image of a breast M by irradiating the breast M of the subject as a target with radiations R (for example, X rays) from a radiation source 29.

The mammography apparatus 10 has a function of performing normal imaging in which imaging is performed in a state where the radiation source 29 is positioned at an irradiation position along a normal direction of a detection surface 20A of a radiation detector 20 and a function of performing tomosynthesis imaging in which imaging is performed in a state where the radiation source 29 is moved to each of a plurality of irradiation positions.

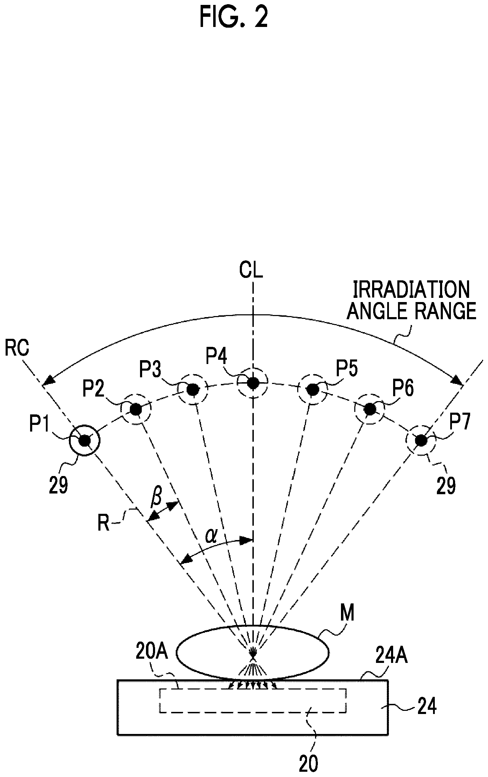

As illustrated in Fig. 1, the mammography apparatus 10 includes an imaging table 24, a base 26, an arm portion 28, and a compression unit 32. A radiation detector 20 is disposed inside the imaging table 24. As illustrated in Fig. 2, in the mammography apparatus 10, in a case of performing imaging, the breast M of the subject is positioned on an imaging surface 24A of the imaging table 24 by a user.

The radiation detector 20 detects radiations R passing through the breast M as a target. Specifically, the radiation detector 20 detects the radiations R that pass through the breast M of the subject, enter into the imaging table 24, and reach a detection surface 20A of the radiation detector 20, and generates a radiation image based on the detected radiations R. The radiation detector 20 outputs image data representing the generated radiation image. In the following, a series of operations of irradiating the breast with radiations R from the radiation source 29 and generating a radiation image by the radiation detector 20 may be referred to as "imaging". The radiation detector 20 may be an indirect-conversion-type radiation detector that converts the radiations R into light beams and converts the converted light beams into charges, or may be a direct-conversion-type radiation detector that directly converts the radiations R into charges.

A compression plate 30 that is used for compressing the breast M when performing imaging is attached to the compression unit 32. The compression plate 30 is moved in a direction toward or away from the imaging table 24 (hereinafter, referred to as a "vertical direction") by a compression plate driving unit (not illustrated) provided in the compression unit 32. The compression plate 30 compresses the breast M between the compression plate 30 and the imaging table 24 by moving in the vertical direction.

The arm portion 28 can be rotated with respect to the base 26 by a shaft portion 27. The shaft portion 27 is fixed to the base 26, and the shaft portion 27 and the arm portion 28 are rotated as one body. Gears are provided in each of the shaft portion 27 and the compression unit 32 of the imaging table 24. By switching the gears between an engaged state and a non-engaged state, the compression unit 32 of the imaging table 24 and the shaft portion 27 can be switched between a state where the compression unit 32 and the shaft portion 27 are connected to each other and are rotated as one body and a state where the shaft portion 27 is separated from the imaging table 24 and idles. Elements for switching between transmission and non-transmission of power of the shaft portion 27 are not limited to the gears, and various mechanical elements can be used. The arm portion 28 and the imaging table 24 can be separately rotated with respect to the base 26 with the shaft portion 27 as a rotation axis.

In a case of performing tomosynthesis imaging in the mammography apparatus 10, the radiation source 29 is sequentially moved to each of a plurality of irradiation positions having different irradiation angles by rotation of the arm portion 28. The radiation source 29 includes a radiation tube (not illustrated) that generates the radiations R, and the radiation tube is moved to each of the plurality of irradiation positions in accordance with the movement of the radiation source 29.

Fig. 2 illustrates an example of tomosynthesis imaging. In Fig. 2, the compression plate 30 is not illustrated. In the present embodiment, the radiation source 29 is moved to irradiation positions Pk (k = 1, 2, ..., 7) at which irradiation angles are different by a certain angle β. That is, the radiation source 29 is sequentially moved to a plurality of positions at which the irradiation angles of the radiations R with respect to the detection surface 20A of the radiation detector 20 are different. In Fig. 2, the number of the irradiation positions Pk is set to 7. On the other hand, the number of the irradiation positions Pk is not limited and can be changed as appropriate.

At each irradiation position Pk, the radiation R is emitted from the radiation source 29 toward the breast M, and the radiation detector 20 generates a radiation image by detecting the radiation R passing through the breast M. In the radiography system 2, in a case where the radiation source 29 is moved to each of the irradiation positions Pk and tomosynthesis imaging for generating a radiation image at each irradiation position Pk is performed, in the example of Fig. 2, seven radiation images are obtained.

In the following, in the tomosynthesis imaging, the radiation image obtained by performing imaging at each irradiation position Pk is referred to as a "projection image" in a case of distinguishing and describing the radiation image from a tomographic image, and a plurality of projection images obtained by performing tomosynthesis imaging once are referred to as a "series of the plurality of projection images". Further, in a case where the projection image is referred to without distinguishing the projection image from the tomographic image, the projection image is simply referred to as a "radiation image".

In addition, as illustrated in Fig. 2, the irradiation angle of the radiation R means an angle α formed by a normal line CL of the detection surface 20A of the radiation detector 20 and a radiation axis RC. The radiation axis RC means an axis connecting a focus of the radiation source 29 at each irradiation position Pk and a preset position. Further, the detection surface 20A of the radiation detector 20 is a surface substantially parallel to the imaging surface 24A. The radiation R emitted from the radiation source 29 is a cone beam having a focus as the apex and the radiation axis RC as a central axis.

On the other hand, in a case of performing normal imaging in the mammography apparatus 10, the position of the radiation source 29 is fixed to the irradiation position P4 at which the irradiation angle $\alpha$ is 0 degree. The radiation R is emitted from the radiation source 29 according to an instruction of the console 12, and the radiation detector 20 generates a radiation image by detecting the radiation R passing through the breast M.

The mammography apparatus 10 and the console 12 are connected to each other by wired communication or wireless communication. The radiation image generated by the radiation detector 20 in the mammography apparatus 10 is output to the console 12 by wired communication or wireless communication via a communication interface (I/F) (not illustrated).

The console 12 includes a controller 40, a storage unit 42, a user I/F 44, and a communication I/F 46. As described above, the controller 40 has a function of performing control related to radiography by the mammography apparatus 10. The controller 40 is configured with, for example, a computer system including a central processing unit (CPU), a read only memory (ROM), and a random access memory (RAM).

The storage unit 42 stores information related to radiography, the radiation image acquired from the mammography apparatus 10, and the like. The storage unit 42 is a non-volatile storage such as a hard disk drive (HDD) or a solid state drive (SSD).

The user I/F 44 includes an input device including various buttons and switches, which are related to imaging of the radiation image and are operated by a user such as a technician, and a lamp, a display, or the like that displays information related to imaging, the radiation image obtained by imaging, and the like.

The communication I/F 46 performs communication of various types of data such as the information related to radiography, the radiation image, and the like between the console 12 and the mammography apparatus 10 by wired communication or wireless communication. Further, the communication I/F 46 performs communication of various types of data such as the radiation image between the PACS 14 and the image processing apparatus 16 via the network 17 by wired communication or wireless communication.

In addition, the PACS 14 includes a storage unit 50 (refer to Fig. 1) that stores a radiation image group 52. The radiation image group 52 includes a projection image acquired from the console 12 via the network 17.

The image processing apparatus 16 has a function of supporting diagnosis by a doctor by performing determination related to diagnosis of a lesion in a case where a doctor or the like (hereinafter, simply referred to as a "doctor") performs diagnosis related to a lesion of the breast M using the radiation image.

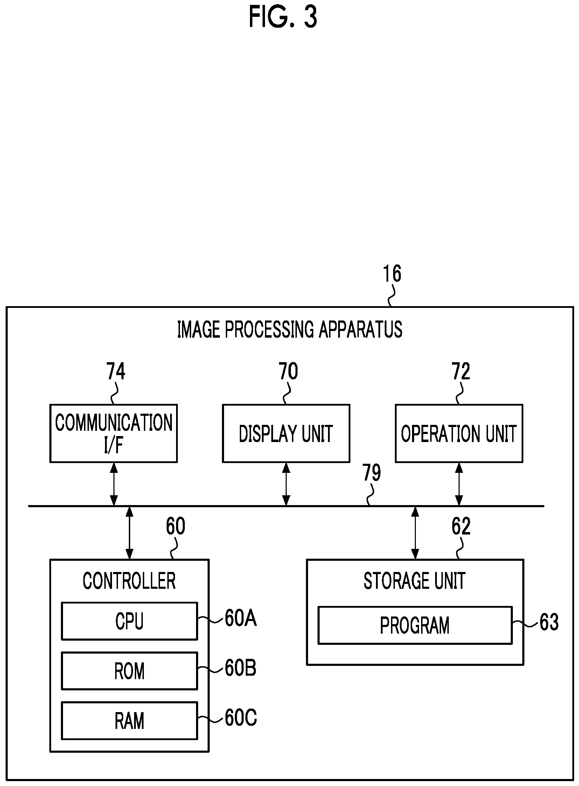

Fig. 3 illustrates an example of a configuration of the image processing apparatus 16. The image processing apparatus 16 includes a controller 60, a storage unit 62, a display unit 70, an operation unit 72, and a communication I/F 74. The controller 60, the storage unit 62, the display unit 70, the operation unit 72, and the communication I/F 74 are connected to each other via a bus 79 such as a system bus or a control bus such that various types of information can be exchanged.

The controller 60 controls overall operations of the image processing apparatus 16. The controller 60 is configured with a computer system including a CPU 60A, a ROM 60B, and a RAM 60C. Various programs, data, and the like for performing control by the CPU 60A are stored in advance in the ROM 60B. The RAM 60C temporarily stores various types of data.

The storage unit 62 is a non-volatile storage such as an HDD or an SSD. The storage unit 62 stores a program 63 or the like for causing the controller 60 to execute various processing.

The display unit 70 is a display that displays a radiation image, various types of information, and the like. The operation unit 72 is used to allow a doctor to input an instruction for diagnosing a lesion of a breast using a radiation image, various types of information, and the like. The operation unit 72 includes, for example, various switches, a touch panel, a touch pen, a mouse, and the like.

The communication I/F 74 performs communication of various types of information between the console 12 and the PACS 14 via the network 17 by wireless communication or wired communication.

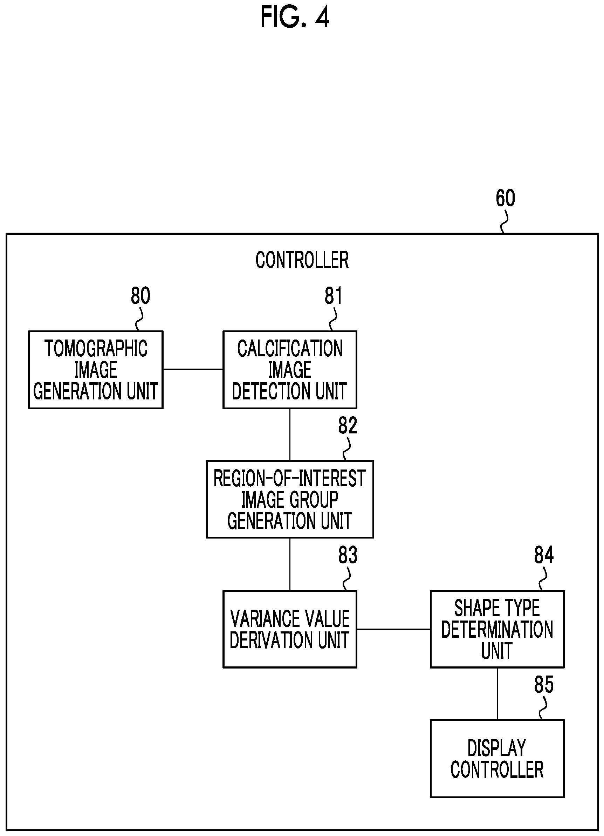

Fig. 4 illustrates an example of a function realized by the controller 60 of the image processing apparatus 16. The CPU 60A of the controller 60 realizes various functions by executing processing based on the program 63 stored in the storage unit 62. The controller 60 functions as a tomographic image generation unit 80, a calcification image detection unit 81, a region-of-interest image group generation unit 82, a variance value derivation unit 83, a shape type determination unit 84, and a display controller 85.

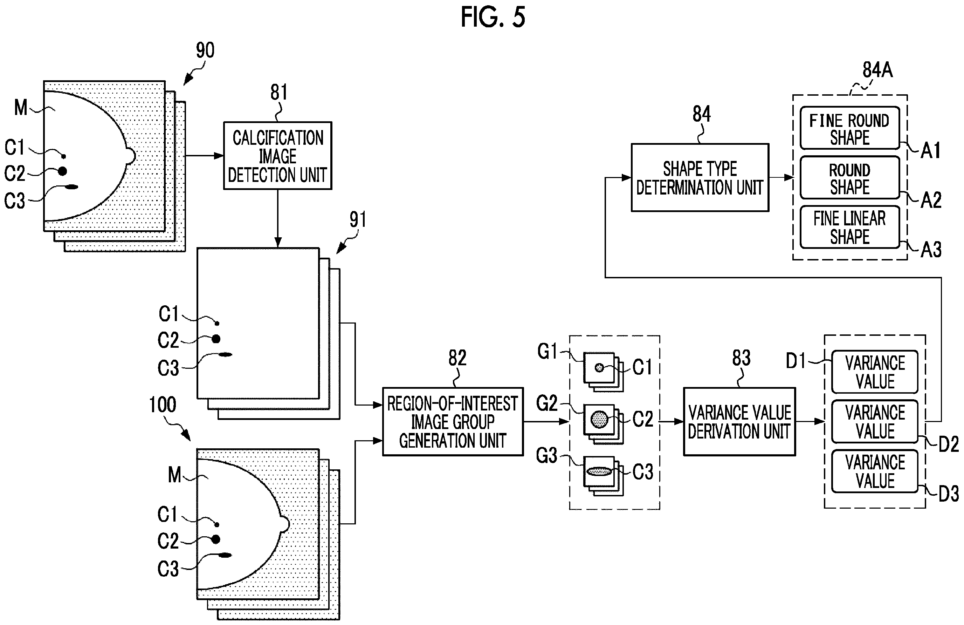

The tomographic image generation unit 80 has a function of generating a plurality of tomographic images 90 (refer to Fig. 5) from a series of the plurality of projection images 100. The tomographic image generation unit 80 acquires a series of the plurality of projection images 100 from the console 12 of the mammography apparatus 10 or the PACS 14 based on an instruction for diagnosing a lesion. The tomographic image generation unit 80 generates a plurality of tomographic images 90 having different heights from the imaging surface 24A, from a series of the plurality of acquired projection images 100. For example, the tomographic image generation unit 80 generates a plurality of tomographic images 90 by reconfiguring a series of the plurality of projection images 100 by a back projection method. As the back projection method, a filter back projection (FBP) method, a successive approximation reconfiguration method, or the like can be used. The tomographic image generation unit 80 outputs the plurality of generated tomographic images 90 to the calcification image detection unit 81.

Fig. 5 schematically illustrates a flow of processing by the image processing apparatus 16. Processing by the calcification image detection unit 81, the region-of-interest image group generation unit 82, the variance value derivation unit 83, and the shape type determination unit 84 will be described with reference to Fig. 5.

The calcification image detection unit 81 performs calcification image detection processing of detecting a tissue image in which an occurrence of calcification is expected in the breast M (hereinafter, calcification image) based on the plurality of tomographic images 90 generated by the tomographic image generation unit 80. As the calcification image detection unit 81, a detector using a known computer-aided diagnosis (CAD) algorithm can be used. In the CAD algorithm, a probability (likelihood) indicating that a pixel in the tomographic image 90 is a calcification image is derived, and a pixel of which the probability is equal to or higher than a predetermined threshold value is detected as the calcification image.

The calcification image detection unit 81 is not limited to the detector using the CAD algorithm, and may be configured by a machine-learned model obtained by performing machine learning.

The detection result of the calcification image by the calcification image detection unit 81 is output as, for example, a plurality of mask images 91 each of which represents a position of the calcification image. Each of the plurality of mask images 91 is a binary image in which a pixel included in the calcification image is represented by "1" and the other pixels are represented by "0". The calcification image detection unit 81 outputs the plurality of mask images 91 corresponding to each of the plurality of tomographic images 90. By performing the detection processing using the plurality of tomographic images 90, the calcification image can be detected with high detection accuracy. In the example illustrated in Fig. 5, three calcification images C1 to C3 are detected by the calcification image detection unit 81.

The region-of-interest image group generation unit 82 performs region-of-interest image group generation processing of generating a region-of-interest image group (hereinafter, referred to as a ROI (region of interest) image group) based on a series of the plurality of projection images 100 used for reconfiguration processing by the tomographic image generation unit 80, a detection result of the calcification image by the calcification image detection unit 81, and position information of the radiation tube at a time when imaging each of a series of the plurality of projection images 100.

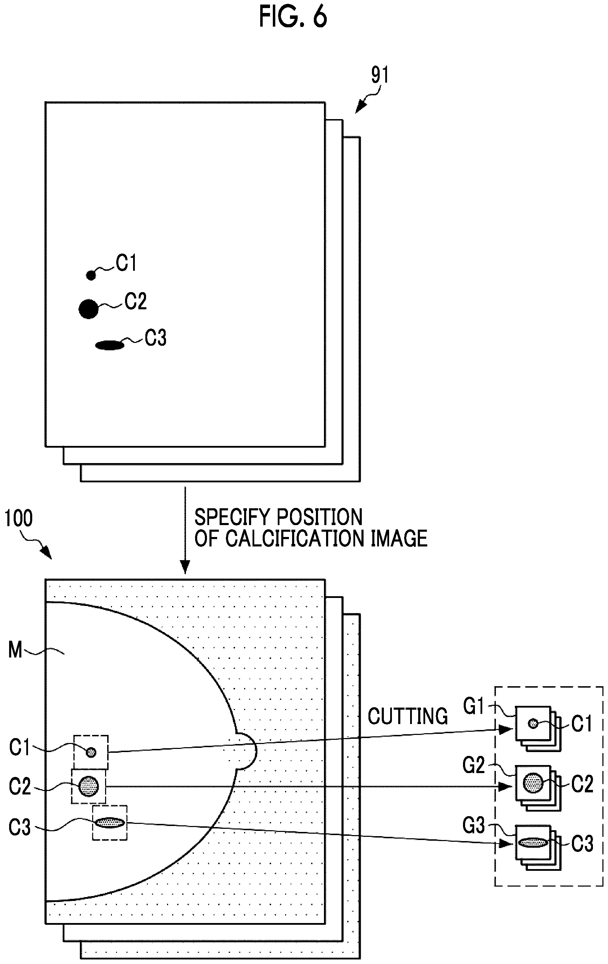

Fig. 6 conceptually illustrates an example of region-of-interest image group generation processing by the region-of-interest image group generation unit 82. The region-of-interest image group generation unit 82 generates a ROI image group including a plurality of ROI images by cutting out, as a ROI image, a region including a calcification image from each of a series of the plurality of projection images 100 based on the plurality of mask images 91. In addition, in a case where the plurality of calcification images are detected in the calcification image detection processing, the region-of-interest image group generation unit 82 individually generates a ROI image group for each of the plurality of calcification images. In the example illustrated in Fig. 6, a ROI image group is individually generated for each of three calcification images C1 to C3. Thereby, a ROI image group G1 including the calcification image C1, a ROI image group G2 including the calcification image C2, and a ROI image group G3 including the calcification image C3 are generated.

The variance value derivation unit 83 performs variance value calculation processing of calculating a variance value of feature amounts of each of the ROI images included in the ROI image group.

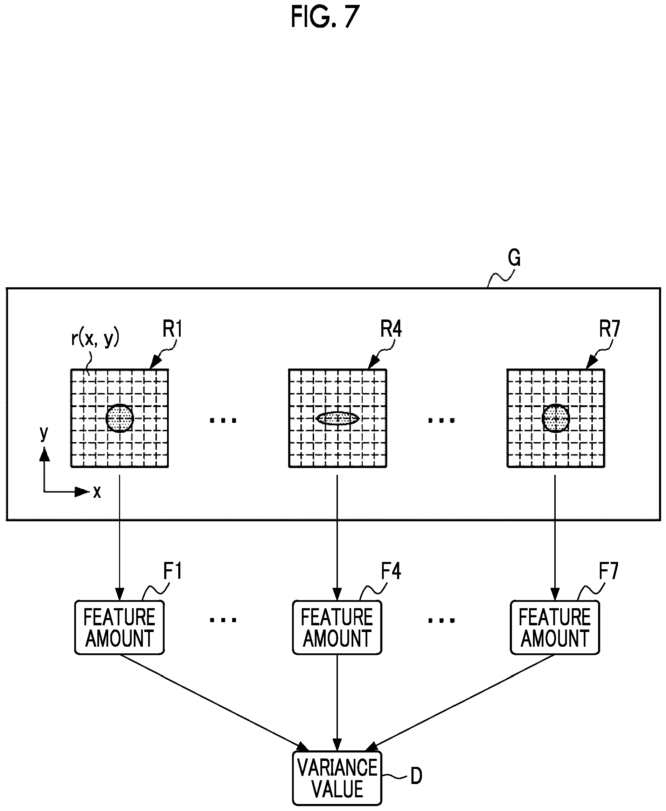

Fig. 7 conceptually illustrates an example of variance value calculation processing by the variance value derivation unit 83. Specifically, Fig. 7 illustrates variance value calculation processing for one ROI image group G. The ROI image group G includes seven ROI images R1 to R7. The ROI image Rk is an image cut out from the projection image acquired at the irradiation position Pk. First, the variance value derivation unit 83 calculates a feature amount Fk of the ROI image Rk based on the following Equation (1).

$$ \mathrm{Fk} = \frac{1}{\mathrm{n}} \sum_{x,y \in \mathrm{Rk}} (\mathrm{r}(x,y) - \mathrm{r}_a)^2 \quad \cdots (1) $$

Here, r(x, y) is a pixel value of a pixel at a coordinate (x, y) in the ROI image Rk. $r_a$ is an average value of pixel values r(x, y) included in the ROI image Rk. In addition, n is the number of pixels included in the ROI image Rk.

In the present embodiment, the feature amount Fk is a variance value of the pixel values r(x, y) included in the ROI image Rk. Seven feature amounts F1 to F7 are calculated from the seven ROI images R1 to R7. It is considered that more pixels (for example, high-brightness pixels) corresponding to the calcification image are included in the ROI image Rk as the feature amount Fk is larger.

In addition, the variance value derivation unit 83 calculates a variance value D based on the following Equation (2).

$$ \mathrm{D} = \frac{1}{7} \sum_{k=1}^{7} (\mathrm{Fk} - \mathrm{F}_a)^2 \quad \cdots (2) $$

Here, $F_a$ is an average value of the feature amounts F1 to F7. The variance value D is a value representing a degree of variation of the feature amounts F1 to F7. As the variance value D is larger, a change in shape due to a difference in the irradiation position Pk is larger. For example, as the variance value D is smaller, the shape of the calcification image is closer to a circle shape. As the variance value D is larger, the shape of the calcification image is closer to a linear shape.

In the example illustrated in Fig. 5, the variance value derivation unit 83 outputs the variance values D1 to D3 generated based on the ROI image groups G1 to G3 to the shape type determination unit 84.

The shape type determination unit 84 performs shape type determination processing of determining a type of a shape of the calcification image based on the variance value calculated by the variance value derivation unit 83. The shape type determination unit 84 holds information in which a relationship between a predetermined variance value and a type of a shape of the calcification image is defined, and determines a type of a shape of the calcification image based on the information. That is, the shape type determination unit 84 is a rule-based determination model based on a relationship between a predetermined variance value and a type of a shape of the calcification image.

In the example illustrated in Fig. 5, the shape type determination unit 84 determines a type of a shape of each of the calcification images C1 to C3 included in the ROI image groups G1 to G3 based on the variance values D1 to D3. The shape type determination unit 84 outputs a shape type determination result 84A indicating the type of the shape of the calcification image. The shape type determination result 84A includes determination results A1 to A3. The determination result A1 represents that a type of a shape of the calcification image C1 is "fine round shape". The determination result A2 represents that a type of a shape of the calcification image C2 is "round shape". The determination result A3 represents that a type of a shape of the calcification image C3 is "fine linear shape".

In the present embodiment, the type of the shape of the calcification image includes not only a difference in shape but also a difference in size. The type of the shape of the calcification image is not limited to the above-described example. Preferably, the type of the shape of the calcification image is classified into classes such that whether the calcification image represents benignancy or malignancy can be determined.

In addition, the shape type determination unit 84 may perform shape type determination processing using a machine-learned model obtained by performing machine learning of the relationship between the variance value and the type of the shape of the calcification image.

The display controller 85 performs display processing for displaying the shape type determination result 84A by the shape type determination processing on the display unit 70. Specifically, the display controller 85 highlights and displays the calcification image having a specific shape based on the shape type determination result 84A.

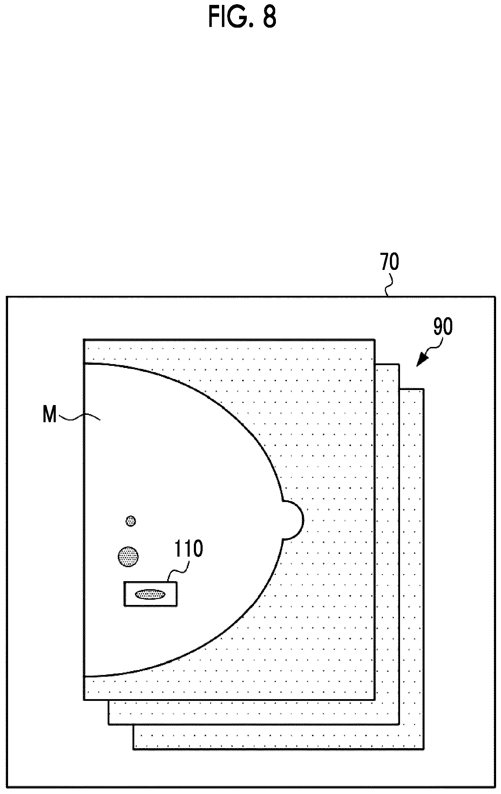

Fig. 8 illustrates an example of display processing by the display controller 85. For example, the display controller 85 displays the shape type determination result 84A together with the tomographic image 90 as a clinical image on the display unit 70. In the example illustrated in Fig. 8, the display controller 85 highlights and displays the calcification image having a shape (for example, a linear shape) representing a high degree of malignancy based on the shape type determination result 84A by surrounding the calcification image with a frame 110. The displayed tomographic image 90 with the frame 110 is, for example, one tomographic image 90 selected from the plurality of tomographic images 90 via the operation unit 72.

In the example illustrated in Fig. 8, only the calcification image having a shape representing a high degree of malignancy is surrounded by the frame 110. On the other hand, all the calcification images may be surrounded by frames 110. In this case, colors of the frames 110, line types of the frames 110, and the like may be different based on the shape type determination result 84A. For example, the frame 110 surrounding the calcification image having a shape representing a high degree of malignancy is red, and the frame 110 surrounding the calcification image having a shape representing a low degree of malignancy is blue.

In addition, the highlight display is not limited to the form in which the calcification image is surrounded by the frame 110. The color of the calcification image may be different based on the shape type determination result 84A. For example, the highlight display may be performed by coloring only the calcification image having a shape representing a high degree of malignancy. In addition, texts, symbols, and the like representing the shape type determination result 84A may be displayed on the display unit 70.

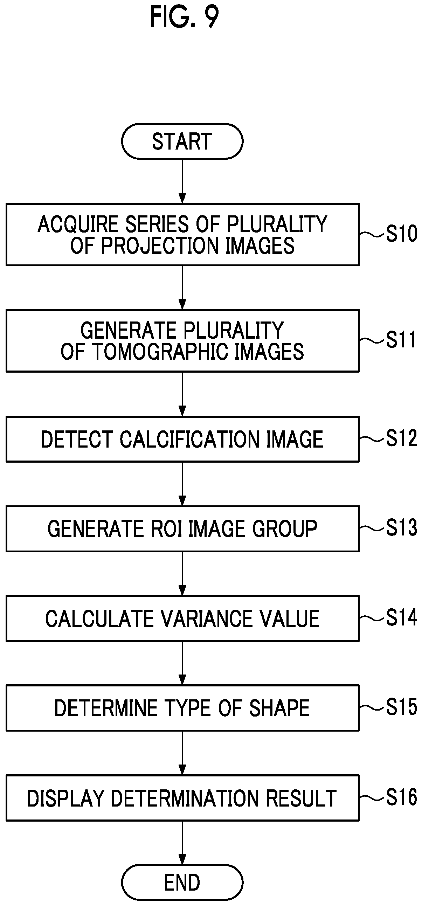

Next, a series of processing by the image processing apparatus 16 will be described with reference to Fig. 9. First, in step S10, the tomographic image generation unit 80 acquires a series of a plurality of projection images 100 from the console 12 of the mammography apparatus 10 or the PACS 14.

In step S11, the tomographic image generation unit 80 generates a plurality of tomographic images 90 based on a series of the plurality of projection images 100 acquired in step S10.

In step S12, the calcification image detection unit 81 detects a calcification image from the plurality of tomographic images 90 generated in step S11, and generates a plurality of mask images 91 as a detection result.

In step S13, the region-of-interest image group generation unit 82 generates a ROI image group by cutting out, as a ROI image, a region including a calcification image from each of a series of the plurality of projection images 100 by using the plurality of mask images 91 generated in step S12.

In step S14, the variance value derivation unit 83 calculates a variance value of feature amounts of each of the ROI images included in the ROI image group generated in step S13.

In step S15, the shape type determination unit 84 determines a type of a shape of the calcification image based on the variance value calculated in step S14, and outputs a shape type determination result 84A.

In step S16, the display controller 85 performs display processing of displaying the shape type determination result 84A obtained in step S15 on the display unit 70. Specifically, the display controller 85 highlights and displays the calcification image having a shape representing a high degree of malignancy.

Generally, in the synthesized two-dimensional image generated from the plurality of tomographic images, a shape of the calcification image is not accurately represented in many cases. Further, in the tomographic image, the calcification image is blurred due to noise and visibility is lowered. On the other hand, according to the technique of the present disclosure, the ROI image group is generated by cutting out a region including the calcification image from each of a series of the plurality of projection images, and the type of the shape of the calcification image is determined based on the variance value of the feature amounts of each of the ROI images included in the ROI image group. Therefore, it is possible to accurately determine the type of the shape of the calcification image.

Further, in the embodiment, the calcification image detection unit 81 detects the calcification image from the plurality of tomographic images 90. The calcification image detection unit 81 may detect only a calcification image (so-called pale calcification image) of which a signal value is equal to or smaller than a certain value. This is because a shape of the pale calcification image is not accurately represented and it is difficult to determine a type of the shape on the tomographic image 90 as a clinical image that is displayed on the display unit 70.

[Modification Example]

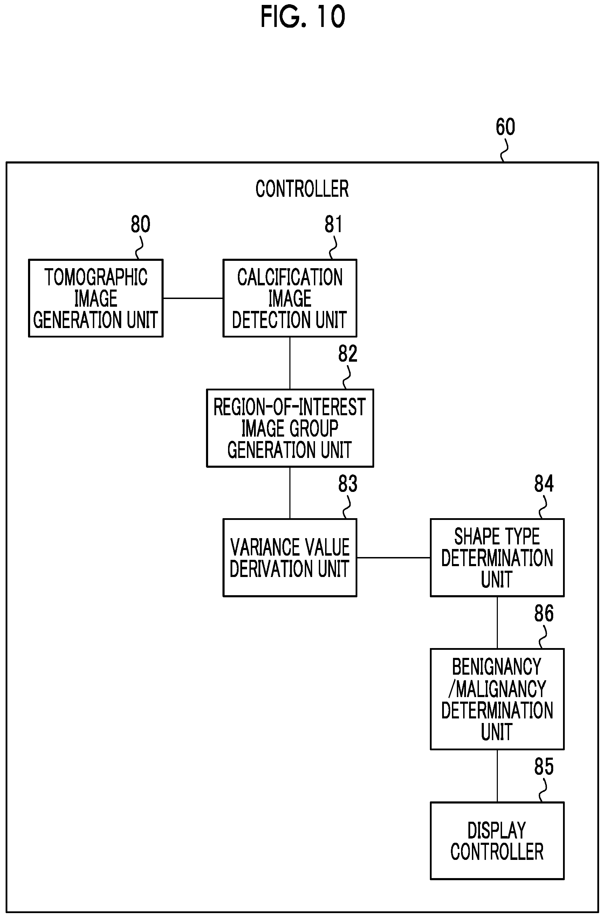

Hereinafter, a modification example of the embodiment will be described. Fig. 10 illustrates a function realized by the controller 60 of the image processing apparatus 16 according to a modification example. The present modification example is different from the embodiment in that the controller 60 functions as a benignancy/malignancy determination unit 86 in addition to the tomographic image generation unit 80, the calcification image detection unit 81, the region-of-interest image group generation unit 82, the variance value derivation unit 83, the shape type determination unit 84, and the display controller 85.

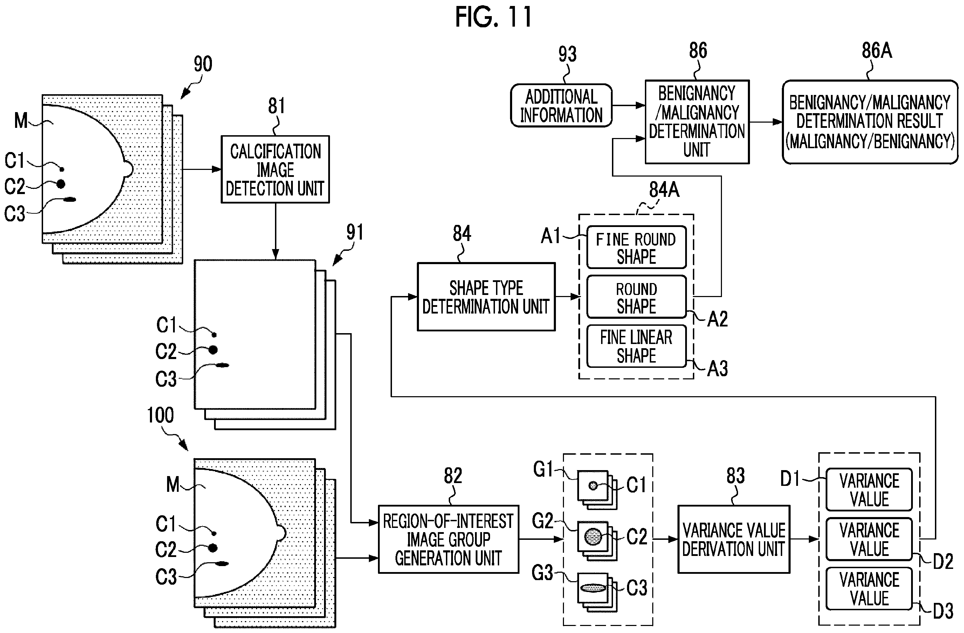

Fig. 11 schematically illustrates a flow of processing by the image processing apparatus 16 according to the modification example. In the present modification example, the shape type determination result 84A output from the shape type determination unit 84 is input to the benignancy/malignancy determination unit 86. The benignancy/malignancy determination unit 86 determines whether the calcification image represents benignancy or malignancy, or determines a degree of malignancy represented by the calcification image, based on the shape type determination result 84A. In the example illustrated in Fig. 11, the benignancy/malignancy determination unit 86 determines whether the calcification image C1 represents benignancy or malignancy based on the determination result A1, determines whether the calcification image C2 represents benignancy or malignancy based on the determination result A2, and determines whether the calcification image C3 represents benignancy or malignancy based on the determination result A3.

In addition, additional information 93 other than the shape type determination result 84A may be input to the benignancy/malignancy determination unit 86. The additional information 93 is, for example, information such as a distribution of the calcification image, the calcification image in the synthesized two-dimensional image, and the like. The benignancy/malignancy determination unit 86 can accurately perform benignancy/malignancy determination by using the additional information 93 in addition to the shape type determination result 84A.

In the present modification example, the display controller 85 performs display processing of displaying the benignancy/malignancy determination result 86A output from the benignancy/malignancy determination unit 86 on the display unit 70. For example, the display controller 85 highlights and displays the calcification image determined as a malignancy based on the benignancy/malignancy determination result 86A. The display controller 85 may display a text, a symbol, or the like representing the benignancy/malignancy determination result 86A on the display unit 70.

As the benignancy/malignancy determination unit 86, for example, a determination device using a known CAD algorithm can be used. The benignancy/malignancy determination unit 86 may be configured by a machine-learned model obtained by performing machine learning.

[Other Modification Examples]

In the embodiment and the modification example, the feature amount Fk calculated by the variance value derivation unit 83 is, as described in Equation (1), a variance value of the pixel values included in the ROI image Rk with respect to the average value $r_a$ of the pixel values included in the ROI image Rk. Instead, the feature amount Fk calculated by the variance value derivation unit 83 may be a variance value of the pixel values included in the ROI image Rk with respect to an average value of pixel values in a region of the breast M (hereinafter, referred to as a breast region) of one projection image among a series of the plurality of projection images 100. That is, the average value $r_a$ in Equation (1) may be an average value of pixel values in a breast region of one projection image (for example, a projection image from which the ROI image Rk is cut out). In this case, the feature amount Fk is represented as a variation in pixel value with respect to a normal value in the breast region.

In addition, the feature amount Fk calculated by the variance value derivation unit 83 may be the number of pixels having a pixel value equal to or larger than a threshold value among a plurality of pixels included in the ROI image Rk. In this case, by setting the threshold value to a lower limit value of values that are allowable for the calcification image, the number of pixels having a pixel value equal to or larger than the threshold value can correspond to the number of pixels included in the calcification image.

The embodiment and the modification examples can be appropriately combined as long as there is no contradiction.

In addition, in the embodiment and the modification examples, as a hardware structure of a processing unit that executes various processing such as the tomographic image generation unit 80, the calcification image detection unit 81, the region-of-interest image group generation unit 82, the variance value derivation unit 83, the shape type determination unit 84, the display controller 85, and the benignancy/malignancy determination unit 86, for example, various processors to be described below can be used. The various processors include a graphics processing unit (GPU) in addition to a CPU. In addition, the various processors are not limited to a general-purpose processor such as a CPU that functions as various processing units by executing software (program), and include a dedicated electric circuit, which is a processor having a circuit configuration specifically designed to execute specific processing, such as a programmable logic device (PLD) or an application specific integrated circuit (ASIC) that is a processor of which the circuit configuration may be changed after manufacturing such as a field programmable gate array (FPGA).

One processing unit may be configured by one of these various processors, or may be configured by a combination of two or more processors of the same type or different types (for example, a combination of a plurality of FPGAs or a combination of a CPU and an FPGA). Further, the plurality of processing units may be configured by one processor.

As an example in which the plurality of processing units are configured by one processor, firstly, as represented by a computer such as a client and a server, a form in which one processor is configured by a combination of one or more CPUs and software and the processor functions as the plurality of processing units may be adopted. Secondly, as represented by a system on chip (SoC) or the like, a form in which a processor that realizes the function of the entire system including the plurality of processing units by one integrated circuit (IC) chip is used may be adopted. As described above, the various processing units are configured by using one or more various processors as a hardware structure.

Further, as the hardware structure of the various processors, more specifically, an electric circuit (circuitry) in which circuit elements such as semiconductor elements are combined may be used.

In addition, in the embodiment and the modification examples, a form in which the program 63 is stored in the storage unit 62 in advance has been described. On the other hand, the present invention is not limited thereto. The program 63 may be provided by being recorded in a non-transitory recording medium such as a compact disc read only memory (CD-ROM), a digital versatile disc read only memory (DVD-ROM), or a Universal Serial Bus (USB) memory. Further, the program 63 may be downloaded from an external apparatus via a network.

The described contents and the illustrated contents are detailed explanations of a part according to the technique of the present disclosure, and are merely examples of the technique of the present disclosure. For example, the descriptions related to the configuration, the function, the operation, and the effect are descriptions related to examples of a configuration, a function, an operation, and an effect of a part according to the technique of the present disclosure. Therefore, it goes without saying that, in the described contents and illustrated contents, unnecessary parts may be deleted, new components may be added, or replacements may be made without departing from the scope of the present invention as defined by the claims. Further, in order to avoid complications and facilitate understanding of the part according to the technique of the present disclosure, in the described contents and illustrated contents, descriptions of technical knowledge and the like that do not require particular explanations to enable implementation of the technique of the present disclosure are omitted.

Explanation of References

    2: radiography system
    10: mammography apparatus
    12: console
    14: PACS
    16: image processing apparatus
    17: network
    20: radiation detector
    20A: detection surface
    24: imaging table
    24A: imaging surface
    26: base
    27: shaft portion
    28: arm portion
    29: radiation source
    30: compression plate
    32: compression unit
    40: controller
    42: storage unit
    44: user I/F
    46: communication I/F

50: storage unit
52: radiation image group
60: controller
62: storage unit
63: program
70: display unit
72: operation unit
74: communication I/F
79: bus
80: tomographic image generation unit
81: calcification image detection unit
82: region-of-interest image group generation unit
83: variance value derivation unit
84: shape type determination unit
84A: shape type determination result
85: display controller
86: benignancy/malignancy determination unit
86A: benignancy/malignancy determination result
90: tomographic image
91: mask image
93: additional information
100: projection image
110: frame
$\alpha$: irradiation angle
$\beta$: angle
A1, A2, A3: determination result
C1, C2, C3: calcification image
CL: normal line
D, D1, D2, D3: variance value
Fk: feature amount
G, G1, G2, G3: ROI image group
M: breast
Pk: irradiation position
Rk: ROI image
R: radiation
RC: radiation axis

**Claims**

1. An image processing apparatus comprising:

    at least one processor (16),
    wherein the processor is configured to execute

        calcification image detection processing of detecting a calcification image based on a plurality of tomographic images obtained from a series of a plurality of projection images obtained by tomosynthesis imaging of a breast,
        region-of-interest image group generation processing of generating a region-of-interest image group by cutting out, as a region-of-interest image, a region including the calcification image detected by the calcification image detection processing from each of the plurality of projection images,

variance value calculation processing of calculating a variance value of feature amounts of each of the region-of-interest images included in the region-of-interest image group, and
shape type determination processing of determining a type of a shape of the calcification image based on the variance value calculated by the variance value calculation processing.

2. The image processing apparatus according to claim 1,
wherein the processor is configured to individually generate the region-of-interest image group for each of a plurality of the calcification images in the region-of-interest image group generation processing in a case where the plurality of calcification images are detected in the calcification image detection processing.

3. The image processing apparatus according to claim 1 or 2,
wherein the processor is configured to detect only the calcification image of which a signal value is equal to or smaller than a certain value in the calcification image detection processing.

4. The image processing apparatus according to any one of claims 1 to 3,
wherein the processor is configured to determine a shape of the calcification image based on a relationship between a predetermined variance value and a type of a shape in the shape type determination processing.

5. The image processing apparatus according to any one of claims 1 to 4,
wherein the feature amount is a variance value of pixel values included in one of the region-of-interest images.

6. The image processing apparatus according to any one of claims 1 to 4,
wherein the feature amount is a variance value of pixel values included in one of the region-of-interest images with respect to an average value of pixel values in a breast region of one of the projection images.

7. The image processing apparatus according to any one of claims 1 to 4,
wherein the feature amount is the number of pixels having a pixel value equal to or larger than a threshold value among a plurality of pixels included in one of the region-of-interest images.

8. The image processing apparatus according to any one of claims 1 to 7,

wherein the processor is configured to further execute display processing of displaying a shape type determination result by the shape type determination processing on a display unit.

9. The image processing apparatus according to claim 8,
wherein the processor is configured to highlight and display the calcification image having a specific shape based on the shape type determination result in the display processing.

10. An image processing method comprising:

a calcification image detection step of detecting a calcification image based on a plurality of tomographic images obtained from a series of a plurality of projection images obtained by tomosynthesis imaging of a breast;
a region-of-interest image group generation step of generating a region-of-interest image group by cutting out, as a region-of-interest image, a region corresponding to the calcification image detected by the calcification image detection step from each of the plurality of projection images;
a variance value calculation step of calculating a variance value of feature amounts of each of the region-of-interest images included in the region-of-interest image group; and
a shape type determination step of determining a type of a shape of the calcification image based on the variance value calculated by the variance value calculation step.

11. A computer-readable storage medium storing a program causing a computer to execute a process comprising:

calcification image detection processing of detecting a calcification image based on a plurality of tomographic images obtained from a series of a plurality of projection images obtained by tomosynthesis imaging of a breast;
region-of-interest image group generation processing of generating a region-of-interest image group by cutting out, as a region-of-interest image, a region corresponding to the calcification image detected by the calcification image detection processing from each of the plurality of projection images;
variance value calculation processing of calculating a variance value of feature amounts of each of the region-of-interest images included in the region-of-interest image group; and
shape type determination processing of determining a type of a shape of the calcification image based on the variance value calculated by the variance value calculation processing.

**Patentansprüche**

1. Bildverarbeitungsgerät aufweisend:

mindestens einen Prozessor (16),
wobei der Prozessor so konfiguriert ist, dass er Folgendes ausführt

Verkalkungsbilderkennungsprozess zum Erkennen eines Verkalkungsbilds auf der Grundlage einer Vielzahl von tomographischen Bildern, die aus einer Serie einer Vielzahl von Projektionsbildern gewonnen werden, die durch Tomosyntheseabbildung einer Brust gewonnen werden,
Bildgruppe-mit-interessantem-Bereich-Erzeugungsprozess zum Erzeugen einer Gruppe von Bildern mit interessantem Bereich durch Ausschneiden eines Bereichs als Bild mit interessantem Bereich, der das Verkalkungsbild enthält, das durch den Verkalkungsbilderkennungsprozess aus jedem der Vielzahl von Projektionsbildern erkannt wurde,
Varianzwertberechnungsprozess zur Berechnung eines Varianzwerts von Merkmalsbeträgen jedes der in der Gruppe der Bilder mit interessantem Bereich enthaltenen Bilder, und
Formtypbestimmungsprozess zur Bestimmung eines Typs einer Form des Verkalkungsbildes auf der Grundlage des durch den Varianzwertberechnungsprozess berechneten Varianzwertes.

2. Bildverarbeitungsgerät nach Anspruch 1,
wobei der Prozessor so konfiguriert ist, dass er die Gruppe von Bildern mit interessantem Bereich für jedes aus einer Vielzahl von Verkalkungsbildern in dem Bildgruppe-mit-interessantem-Bereich-Erzeugungsprozess individuell erzeugt, wenn die Vielzahl von Verkalkungsbildern in dem Verkalkungsbilderkennungsprozess erkannt wird.

3. Bildverarbeitungsgerät nach Anspruch 1 oder 2,
wobei der Prozessor so konfiguriert ist, dass er nur das Verkalkungsbild erkennt, dessen Signalwert gleich oder kleiner als ein bestimmter Wert bei dem Verkalkungsbilderkennungsprozess ist.

4. Bildverarbeitungsgerät nach einem der Ansprüche 1 bis 3,
wobei der Prozessor so konfiguriert ist, dass er eine Form des Verkalkungsbildes auf der Grundlage einer Beziehung zwischen einem vorbestimmten Varianzwert und einem Typ einer Form in dem Formtypbestimmungsprozess bestimmt.

5. Bildverarbeitungsgerät nach einem der Ansprüche 1 bis 4,
wobei der Merkmalsbetrag ein Varianzwert von Pixelwerten ist, die in einem der Bilder mit interessantem Bereich enthalten sind.

6. Bildverarbeitungsgerät nach einem der Ansprüche 1 bis 4,
wobei der Merkmalsbetrag ein Varianzwert von Pixelwerten ist, die in einem der Bilder mit interessantem Bereich in Bezug auf einen Durchschnittswert von Pixelwerten in einem Brustbereich eines der Projektionsbilder enthalten sind.

7. Bildverarbeitungsgerät nach einem der Ansprüche 1 bis 4,
wobei die Merkmalsmenge die Anzahl von Pixeln ist, die einen Pixelwert haben, der gleich oder größer ist als ein Schwellenwert unter einer Vielzahl von Pixeln, die in einem der Bilder mit interessantem Bereich enthalten sind.

8. Bildverarbeitungsgerät nach einem der Ansprüche 1 bis 7,
wobei der Prozessor so konfiguriert ist, dass er ferner ausführt einen Anzeigeprozess zum Anzeigen eines Ergebnisses der Formtypbestimmung durch den Formtypbestimmungsprozess auf einer Anzeigeeinheit.

9. Bildverarbeitungsgerät nach Anspruch 8,
wobei der Prozessor konfiguriert ist zum Hervorheben und Anzeigen des Verkalkungsbilds mit einer bestimmten Form auf der Grundlage des Ergebnisses der Formtypbestimmung in dem Anzeigeprozess.

10. Bildverarbeitungsverfahren, das Folgendes umfasst:

einen Verkalkungsbilderkennungsschritt zur Erkennung von Verkalkungsbildern, bei dem ein Verkalkungsbild auf der Grundlage einer Vielzahl von tomographischen Bildern erkannt wird, die aus einer Serie einer Vielzahl von Projektionsbildern gewonnen werden, die durch Tomosynthese-Bildgebung einer Brust gewonnen werden;
einen Bildergruppe-mit-interessantem-Bereich-Erzeugungsschritt zum Erzeugen einer Gruppe von Bildern mit interessantem Bereich durch Ausschneiden eines Bereichs als Bild mit interessantem Bereich, der dem Verkalkungsbild entspricht, das durch den Verkalkungsbilderkennungsschritt aus jedem der mehreren Projektionsbilder erkannt wurde;
einen Varianzwertberechnungsschritt zum Berechnen eines Varianzwerts von Merkmalsbeträgen jedes der Bilder mit interessantem Bereich, die in der Gruppe der Bilder mit interessantem Bereich enthalten sind; und
einen Formtypbestimmungsschritt zum Bestimmen eines Typs einer Form des Verkalkungsbildes auf der Grundlage des durch den Varianzwertberechnungsschritt berechneten Varianzwertes.

11. Computerlesbares Speichermedium, das ein Programm speichert, das einen Computer veranlasst, einen Prozess auszuführen, der Folgendes umfasst:

Verkalkungsbilderkennungsprozess zum Erkennen eines Verkalkungsbilds auf der Grundlage einer Vielzahl von tomographischen Bildern, die aus einer Serie einer Vielzahl von Projektionsbildern gewonnen werden, die durch Tomosyntheseabbildung einer Brust gewonnen werden,
Bildergruppe-mit-interessantem-Bereich-Erzeugungsprozess zum Erzeugen einer Gruppe von Bildern mit interessantem Bereich durch Ausschneiden eines Bereichs als Bild mit interessantem Bereich, der das Verkalkungsbild enthält, das durch den Verkalkungsbilderkennungsprozess aus jedem der Vielzahl von Projektionsbildern erkannt wurde,
Varianzwertberechnungsprozess zur Berechnung eines Varianzwerts von Merkmalsbeträgen jedes der in der Gruppe der Bilder mit interessantem Bereich enthaltenen Bilder, und
Formtypbestimmungsprozess zur Bestimmung eines Typs einer Form des Verkalkungsbildes auf der Grundlage des durch den Varianzwertberechnungsprozess berechneten Varianzwertes.

**Revendications**

1. Appareil de traitement d'images comprenant :

au moins un processeur (16),
dans lequel le processeur est configuré pour exécuter

traitement de détection d'images de calcification consistant à détecter une image de calcification sur la base d'une pluralité d'images tomographiques obtenues à partir d'une série de plusieurs images de projection obtenues par imagerie de tomosynthèse d'un sein,
traitement de génération d'un groupe d'images de région d'intérêt consistant à générer un groupe d'images de région d'intérêt en découpant, en tant qu'image de région d'intérêt, une région comprenant l'image de calcification détectée par le traitement de détection d'image de calcification à partir de chacune de la pluralité d'images de projection,

traitement de calcul de la valeur de variance consistant à calculer une valeur de variance des quantités de caractéristiques de chacune des images de région d'intérêt incluses dans le groupe d'images de région d'intérêt, et

traitement de détermination du type de forme consistant à déterminer un type de forme de l'image de calcification sur la base de la valeur de variance calculée par le traitement de calcul de la valeur de variance.

2. Appareil de traitement d'images selon la revendication 1,

le processeur est configuré pour générer individuellement le groupe d'images de la région d'intérêt pour chacune des images de calcification dans le traitement de génération du groupe d'images de la région d'intérêt dans le cas où la pluralité d'images de calcification est détectée dans le traitement de détection de l'image de calcification.

3. Appareil de traitement d'images selon la revendication 1 ou 2,

le processeur est configuré pour ne détecter que l'image de calcification dont la valeur du signal est égale ou inférieure à une certaine valeur dans le traitement de détection de l'image de calcification.

4. Appareil de traitement d'images selon l'une des revendications 1 à 3,

le processeur est configuré pour déterminer la forme de l'image de calcification sur la base d'une relation entre une valeur de variance prédéterminée et un type de forme dans le traitement de détermination du type de forme.

5. Appareil de traitement d'images selon l'une des revendications 1 à 4,

la valeur de la caractéristique est une valeur de variance des valeurs de pixels incluses dans l'une des images de la région d'intérêt.

6. Appareil de traitement d'images selon l'une des revendications 1 à 4,

la valeur de la caractéristique est une valeur de variance des valeurs de pixels incluses dans l'une des images de la région d'intérêt par rapport à une valeur moyenne des valeurs de pixels dans une région du sein de l'une des images de projection.

7. Appareil de traitement d'images selon l'une des revendications 1 à 4,

la quantité de caractéristiques est le nombre de pixels dont la valeur est égale ou supérieure à une valeur seuil parmi une pluralité de pixels inclus dans l'une des images de la région d'intérêt.

8. Appareil de traitement d'images selon l'une des revendications 1 à 7,

le processeur est configuré pour exécuter en outre un traitement d'affichage consistant à afficher sur une unité d'affichage le résultat de la détermination du type de forme par le traitement de détermination du type de forme.

9. Appareil de traitement d'images selon la revendication 8,

le processeur est configuré pour mettre en évidence et afficher l'image de calcification ayant une forme spécifique sur la base du résultat de la détermination du type de forme dans le traitement d'affichage.

10. Méthode de traitement d'images comprenant

une étape de détection d'image de calcification consistant à détecter une image de calcification sur la base d'une pluralité d'images tomographiques obtenues à partir d'une série de plusieurs images de projection obtenues par tomosynthèse d'un sein ;

une étape de génération d'un groupe d'images de région d'intérêt consistant à générer un groupe d'images de région d'intérêt en découpant, en tant qu'image de région d'intérêt, une région correspondant à l'image de calcification détectée par l'étape de détection de l'image de calcification dans chacune de la pluralité d'images de projection ;

une étape de calcul de la valeur de variance consistant à calculer une valeur de variance des quantités de caractéristiques de chacune des images de région d'intérêt incluses dans le groupe d'images de région d'intérêt ; et

une étape de détermination du type de forme consistant à déterminer un type de forme de l'image de calcification sur la base de la valeur de variance calculée par l'étape de calcul de la valeur de variance.

11. Support de stockage lisible par ordinateur stockant un programme amenant un ordinateur à exécuter un processus comprenant :

traitement de détection d'images de calcification consistant à détecter une image de calcification sur la base d'une pluralité d'images tomographiques obtenues à partir d'une série de plusieurs images de projection obtenues par imagerie de tomosynthèse d'un sein ;

traitement de génération d'un groupe d'images de région d'intérêt consistant à générer un groupe d'images de région d'intérêt en découpant, en tant qu'image de région d'intérêt, une région correspondant à l'image de calcification détectée par le traitement de détection d'image de calcification à partir de chacune de la pluralité d'images de projection ;

traitement de calcul de la valeur de variance consistant à calculer une valeur de variance des quantités de caractéristiques de chacune des images de région d'intérêt incluses dans le groupe d'images de région d'intérêt ; et

traitement de détermination du type de forme consistant à déterminer un type de forme de l'image de calcification sur la base de la valeur de variance calculée par le traitement de calcul de la valeur de variance.

# FIG. 1

# FIG. 2

## FIG. 3

16

IMAGE PROCESSING APPARATUS

74
COMMUNICATION I/F

70
DISPLAY UNIT

72
OPERATION UNIT

79

60
CONTROLLER

CPU — 60A

ROM — 60B

RAM — 60C

62
STORAGE UNIT

PROGRAM — 63

# FIG. 4

CONTROLLER 60

TOMOGRAPHIC IMAGE GENERATION UNIT 80

CALCIFICATION IMAGE DETECTION UNIT 81

REGION-OF-INTEREST IMAGE GROUP GENERATION UNIT 82

VARIANCE VALUE DERIVATION UNIT 83

SHAPE TYPE DETERMINATION UNIT 84

DISPLAY CONTROLLER 85

FIG. 5

EP 4 215 117 B1

# FIG. 6

SPECIFY POSITION
OF CALCIFICATION IMAGE

CUTTING

# FIG. 7

# FIG. 8

# FIG. 9

```
          ┌─────────────┐
          │    START    │
          └─────────────┘
                 │
                 ▼
    ┌──────────────────────────────┐
    │  ACQUIRE SERIES OF PLURALITY  │───S10
    │     OF PROJECTION IMAGES      │
    └──────────────────────────────┘
                 │
                 ▼
    ┌──────────────────────────────┐
    │      GENERATE PLURALITY       │───S11
    │     OF TOMOGRAPHIC IMAGES     │
    └──────────────────────────────┘
                 │
                 ▼
    ┌──────────────────────────────┐
    │    DETECT CALCIFICATION IMAGE │───S12
    └──────────────────────────────┘
                 │
                 ▼
    ┌──────────────────────────────┐
    │    GENERATE ROI IMAGE GROUP   │───S13
    └──────────────────────────────┘
                 │
                 ▼
    ┌──────────────────────────────┐
    │    CALCULATE VARIANCE VALUE   │───S14
    └──────────────────────────────┘
                 │
                 ▼
    ┌──────────────────────────────┐
    │    DETERMINE TYPE OF SHAPE    │───S15
    └──────────────────────────────┘
                 │
                 ▼
    ┌──────────────────────────────┐
    │  DISPLAY DETERMINATION RESULT │───S16
    └──────────────────────────────┘
                 │
                 ▼
          ┌─────────────┐
          │     END     │
          └─────────────┘
```

# FIG. 10

60

CONTROLLER

80

TOMOGRAPHIC
IMAGE
GENERATION UNIT

81

CALCIFICATION
IMAGE
DETECTION UNIT

82

REGION-OF-INTEREST
IMAGE GROUP
GENERATION UNIT

83

VARIANCE VALUE
DERIVATION UNIT

84

SHAPE TYPE
DETERMINATION
UNIT

86

BENIGNANCY
/MALIGNANCY
DETERMINATION
UNIT

85

DISPLAY
CONTROLLER

FIG. 11

EP 4 215 117 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016022143 A

- JP 2020141867 A

**Non-patent literature cited in the description**

- Micro-Calcification Detection in Digital Tomosynthesis Mammography. **F W WHEELER et al.** PROCEEDINGS OF SPIE. IEEE, 13 February 2006, vol. 6144, 614420-1